# EUROPEAN PATENT SPECIFICATION

(11) **EP 2 819 579 B1**
(45) Date of publication and mention of the grant of the patent: **21.09.2016**
(21) Application number: 13707343.3
(22) Date of filing: 26.02.2013
(51) Int. Cl.: G01N 33/49, G01N 33/52, G01N 33/66, A61B 5/15, A61B 5/155, A61B 5/157

(54) **TEST DEVICE**
TESTVORRICHTUNG
DISPOSITIF DE TEST

(30) Priority: 02.03.2012 GB 201203693
(43) Date of publication of application: 07.01.2015
(73) Proprietor: SmartSensor Telemed Limited, Didcot, Oxfordshire OX11 0QG (GB)
(72) Inventor: JACKSON, James c/o Smartsensor Telemed Limited, Didcot, Oxfordshire OX11 0QG (GB)
(74) Representative: Wilson, Peter David George
(86) International application number: PCT/EP2013/053771
(87) International publication number: WO 2013/127762

(56) References cited:
- EP-A2- 0 199 484
- EP-A2- 1 174 716
- WO-A1-2009/024794
- WO-A2-02/07064
- WO-A2-2007/112034
- US-A1- 2005 096 513
- US-A1- 2010 311 090
- US-A1- 2011 230 743

## Description

This invention relates to devices for testing materials, preferably biological materials, and it relates especially, though not exclusively, to glucose tolerance testing devices, such as (by way of example only) those described in WO-A-2009/024794. Such devices test blood samples and enable patients to make at home, or in other convenient locations, tests which would otherwise require their attendance at a clinic or other medical institution, thereby to develop clinical data which can be displayed by the device and/or forwarded elsewhere for expert clinical analysis.

Devices of the kind described above have particular value to patients lacking ready access to clinical facilities, but the collection of critical evaluation data by patients themselves in domestic or other non-clinical surroundings can compromise the quality of the data.

In such circumstances it is necessary to reduce, so far as is reasonably possible, extraneous factors that might significantly influence the quality of the data resulting from the test.

One such extraneous factor, which is particularly prevalent in territories where these devices can be of greatest use, is humidity and it is an object of this invention to provide test devices of the kind described in which adverse effects of humidity are reduced or eliminated.

According to the invention there is provided a biological test device comprising at least one zone containing biosensor means and/or reagent media and designated to accept a sample of a biological material, wherein said zone is covered until use by a removable humidity resistant cover, characterised in that said device further comprises timing means for recording the time of removal of the or each cover or a time-related index permitting the time of removal to be calculated.

Preferably, said device comprises first and second such zones each containing respective biosensor means and/or reagent media and designated to accept material samples in a predetermined timing sequence, wherein at least the zone designated to accept the later sample is covered until use by a removable humidity resistant cover.

Preferably, respective removable and humidity resistant covers are associated with both of said zones.

In a preferred embodiment of the invention, the or each humidity resistant cover comprises a shaped cover made of a plastics material and adapted to be slidably attached to and removed from the device.

The shaped covers are preferably made by injection moulding.

Preferably, the or each cover incorporates a dessicant material.

In an alternative configuration, the or each cover comprises a peel-off strip of material which is self-adhesively secured to the device so as to temporarily cover its respective zone.

In any event, it is preferred that the or each cover is adherent not only over its respective zone, but also over an adjoining portion of the device so as to cover, until removed, certain procedural instructions relating to the conduct of the test.

It is further preferred that a cover is configured to interact with actuator means associated with electronic components of said device to place the device into an operational, or fully operational, state and/or to cancel an alarm signalling an action point in the test procedure. In a particularly preferred arrangement, the removal of said cover turns the device on or alters the operational state of the device, for example from a low power-consumption "stand-by" mode to a fully operational mode in which the device consumes more power. It is further preferred that the time of removal of the cover is recorded by electronic means within the device.

In further preferred embodiments of the invention, the device includes electronic means for recording time and for sensing and recording temperature and/or humidity, whereby results that may be compromised due to over-exposure of the device to excessive heat and/or humidity can be identified and assessed accordingly.

Typically, the biological material may be blood, saliva or urine, but the actual materials sensed are not critical to the use of the invention.

In order that the invention may be clearly understood and readily carried into effect, one embodiment of the invention will now be described, by way of example only, with reference to the accompanying drawings of which:
Figure 1 shows a test device in accordance with one example of the invention packaged for storage until use, and with anti-humidity covers provided over sensor zones thereof;
Figure 2 shows in more detail the test device of Figure 1 with its anti-humidity covers removed as for use; and
Figure 3 shows schematically, and by way of example only, a typical operating sequence for devices of the general kind described with reference to Figures 1 and 2.

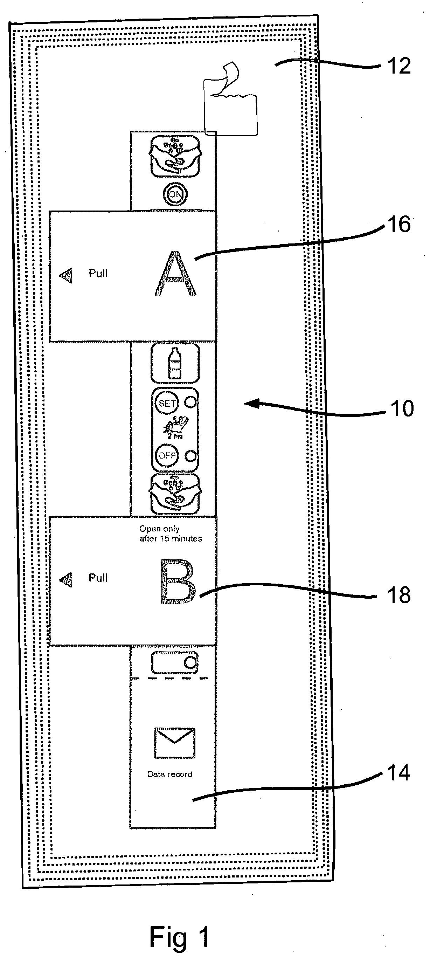

Referring now to Figure 1, a test device 10 in accordance with one example of the invention is packaged for storage within an hermetically sealed pouch 12. If desired, a dessicant medium may be included in the pouch, either as a separate inclusion or incorporated into the envelope 12.

The simple, strip-like form of the device 10 is exemplary only; the requirement for the device being that it presents the various essential stages of the test in a logical sequence that can be readily followed by patients without external guidance. To this end, there is provided on the device a series of graphic images which show the actions to be carried out at each stage of the sequence. Written instructions are also provided, but it is intended that the graphics provide easily understood immediate and topical reference data for patient reassurance and guidance.

The fundamental procedure of the test involves the patient taking a blood sample, for example by way of a finger-prick as is common with routine, day-to-day diabetic testing, both before and after injesting a glycaemic load, typically a glucose drink, thereby to test the patient's tolerance to glucose.

The two blood samples are applied to different sensors in a reasonably strict timing sequence; the sensors developing respective electrical signals that are sent to a data logging area 14 of the device 10, where the signals can be stored as they are, and/or with further processing carried out on them, depending upon the amount of electronic processing incorporated into the device 10. When the process is complete, the device 10 as a whole, or alternatively just the removable data log 14, can be sent or collected for analysis of the test results recorded therein. Alternatively, or in addition, the results may be displayed on the device 10 in a manner suitable for ready interpretation by or for the person undergoing the test.

As can be seen in Figure 1, and as will be more fully described hereinafter, the strip-like device 10 as packaged is, in this example, fitted with two removable covers 16 and 18, each covering a respective one of the two sensors. In this example, the covers 16 and 18 are injection moulded in plastics material and are configured to be a sliding fit on the strip-like device 10. In some embodiments of the invention, the covers are dimensioned and shaped to fit tightly over the device, but they need not be so configured, as it has been found on testing that even a loosely fitted cover can provide the device 10 with significant protection against exposure to high humidity.

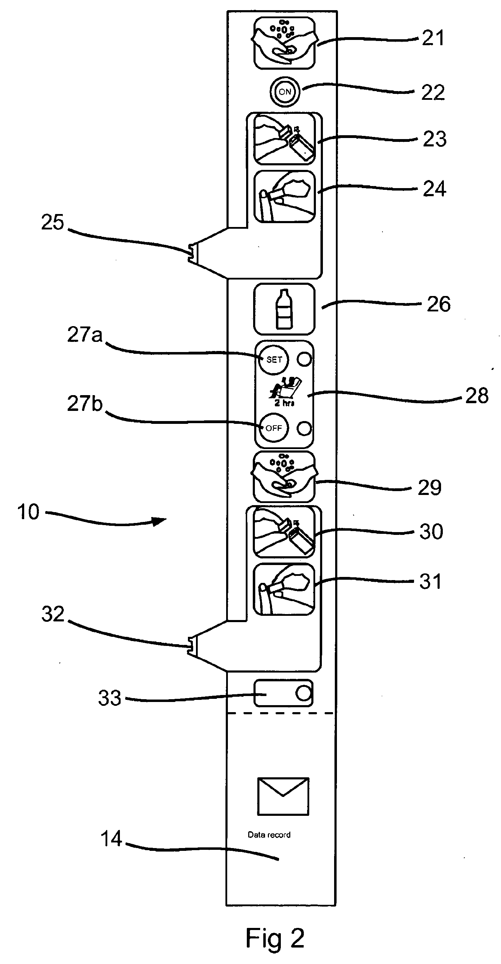

Referring now to Figure 2, the strip-like device 10 is made of any convenient self-supporting material, but it is preferably of plastics material since it is relatively easy to produce reliably and repeatedly in volume; however, non-plastic, water-impermeable or water resistant sheet material can be used if preferred. The device 10 may bear easily legible images, as the graphics displayed thereon perform, as has been mentioned, an important function in guiding the patient through the procedure. Alternatively, the device may be provided with an insert or attachment of material which carries the graphics.

In any event, in the present example, a first graphic 21 indicates to the patient that the hands should be washed and dried before starting the procedure. The patient then actuates a switch device 22 to place the device 10 in a fully operational state. Prior to the actuation of the switch device 22, the device 10 may be completely switched off, or its circuitry may be switched to a stand-by mode in which it consumes little power.

The first cover 16 is then removed, revealing graphics 23, 24 designed to guide the patient through the first finger-prick to produce a first blood sample to be dropped on to a receptor 25 which is configured in known manner to convey the blood to a first sensor (not shown); the receptor and its associated sensor constituting a first test zone. The cover 16 of course, importantly, covers the receptor 25 until it needs to be exposed to receive the blood sample, thereby protecting the associated sensor against the possible effects of humidity after the device 10 has been removed from the pouch 12. It is preferred, though not necessary, for the cover 16 to cover the graphics 23 and 24 as well as the receptor 25 and its associated sensor, as this expedient reduces the amount of instructional data to which the patient is initially exposed and thus assists the patient to take on board the necessary operations.

Preferably, the cover 16 is configured so that its removal either actuates the switch 22, or otherwise automatically places the device 10 in a fully operational state; thereby providing a fail-safe mode of operation, in case the patient should fail to actuate the switch 22.

In such an embodiment, the switch device 22 may be a spring-loaded, mechanically operated switch that is normally held open by the presence of the cover 16, but is closed upon withdrawal of the cover 16. Alternatively, for example, it may comprise a light-sensitive device and the cover 16, or at least the part thereof that covers the switch device 22 when fitted to the strip-like device 10, may be made of or coloured with an opaque substance such that the switch device 22 is only exposed to light when the cover 16 is withdrawn therefrom. In general, it will be appreciated that the switch device 22 may be of any convenient kind and its interaction with the cover 16 can be achieved in any convenient way.

In further alternative arrangements, the cover 16 need not actuate the switch 22, but may be configured to replicate its effect automatically, by suitable interconnection means communicating directly with the electronics carried by the device 10.

Proceeding further with the present example, the patient is then instructed, by way of graphic 26, to injest a prescribed glycaemic load, typically a glucose drink which is provided in a suitable vessel, and then presses a button 27a to start a fixed time period of (in this example) 15 minutes. Other periods can clearly be used if desired or if necessary given differing test conditions and criteria; and in some embodiments the preferred time period is 120 minutes. Graphic 28 instructs the patient to relax until the device 10 produces an audio and/or visual warning that the relevant time period has elapsed and it is necessary to complete the test procedure.

At this point, the patient is, in this example though not necessarily, instructed to press a second button 27b which cancels the timer and switches off the warning that indicates the end of the relaxation period.

The second cover 18 is then removed, exposing graphics 29, 30 and 31 which replicate the graphics 21, 23 and 24 respectively, and which if followed result in the patient applying a second blood sample to a receptor 32, associated with a second sensor, which has remained covered by the cover 18 throughout the procedure to date. The cover 18 can be configured so that its removal either actuates the switch 27b, or otherwise automatically cancels the alarm should the patient not already have done so. In some embodiments of the invention, the button 27b is omitted, and its function replicated automatically in response to removal of the cover 18.

The receptor 32 and its associated sensor constitute a second test zone.

Once the second sample has been applied to the receptor 32, and the necessary electronic operations have been carried out, the results of the test are transferred to electronic storage and/or processing means in the data logging area 14; the successful transfer and thus completion of the test procedure being indicated by the illumination of a lamp device 33, such as an LED. Successful completion of the procedure may also, or instead, be confirmed audibly.

The data logging area 14 is then preferably detached from the remainder of the device 10 and forwarded to an analysis station such as a clinic or other medical centre. In more sophisticated arrangements, the device 10 may be provided with means to calculate the results of the test and to display them, for example on an on-board LCD (not shown), in a readily interpretable manner. Alternatively, or in addition, the data logging section 14 may be fitted with a suitable connector, such as a USB connector, allowing the clinical data resulting from the procedure to be up-loaded via a computer or another suitable electronic device for local display and/or for direct transmission to an analysis centre.

In the above described arrangement, the switch 22 is intended to be actuated by the patient to place the electronic circuitry in the device 10 into its fully operational state but, if this is not done, the removal of cover 16 automatically achieves the same result as a back-up. In some preferred embodiments of the invention, however, the switch 22 is omitted and the removal of cover 16 is effective to automatically place the device 10 into its fully operational state and in preparation for the addition of the first sample to the receptor 25.

Preferably, in any event, the electronics within the device 10 include means to record either the time of removal of the covers 16 and 18 or a time-related index permitting the time of removal to be calculated. This provides a facility whereby a test relating to a sample applied to the receptor 25 too long after the removal of the cover 16, or to the receptor 32 too long after the removal of the cover 18, can be rejected if it appears that the results may have been compromised.

The stability of the sensors associated with the receptors 25 and 32 is influenced not only by humidity, but also by other factors such as temperature. It is thus preferred that the device 10 incorporates at least a temperature sensor, and optionally a humidity sensor too. This facility enables the temperature and possibly also humidity to be recorded, for correlation with the times of application of the samples to the receptors 25 and 32; which times are logged automatically, and can be cross-referenced to the times of removal of the covers 16 and 18. On subsequent review of the data recorded by device 10, or of test results derived from such data, the test may be invalidated if it is found that the sensors in the device have been exposed to certain levels of temperature and/or humidity for certain periods of time. All such assessments, however, are preferably made individually, and it is not intended that invalidations should be made on the basis of predetermined exclusion criteria.

The covers 16 and 18 may each contain or house a dessicant material, in addition to any dessicant that may be included in the original packaging.

As previously mentioned, it is highly desirable for the arrangement to be such that removal of the covers 16 and 18 from the device 10 actuates a component that logs either the precise times of their removal or a time-related index permitting the time of removal to be calculated.

In some embodiments of the invention the rigid, slide on/slide off, covers 16, 18 may be replaced by peel-off covers which may comprise a plurality of layers, at least one of which may be a dessicant layer.

In some embodiments of the invention, the cover 16 associated with the first test zone comprising blood receptor 25 and its associated sensor may be omitted on the expectation that the pouch 12 is not opened until immediately prior to the commencement of the test procedure, and therefore protection from moisture may not be required for the receptor 25 whereas such protection may remain a requirement for the receptor 32.

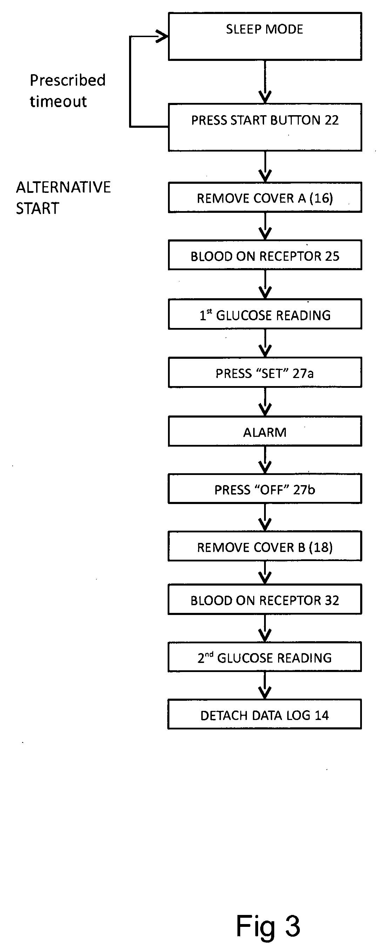

The blocks representing the various steps in the sequence of operations represented schematically, and by way of example only, in Figure 3 bear legends which are self-explanatory in the light of the preceding description. However, in addition and where appropriate, the blocks also carry for the reader's convenience reference numbers consistent with those used in Figures 1 and 2.

The device 10 typically contains an on-board microcontroller (not shown) which is conditioned to receive inputs from the sensors associated with receptors 25 and 32; from the switches such as 22, 27a and 27b and/or means detecting the removal of covers 16 and 18; from environmental sensors such as temperature and/or humidity sensors; and from an RFID transceiver. The microcontroller provides outputs operating, among other things, warning or guidance LEDs and sounders used at appropriate times to prompt or warn a user; and also provides outputs when appropriate to the transceiver.

## Claims

1. A biological test device (10) comprising at least one zone containing biosensor means and/or reagent media and designated to accept a sample of a biological material, **CHARACTERISED IN THAT**
said zone is covered until use by a removable humidity resistant cover (16, 18); and
said device further comprises timing means for recording the time of removal of the or each cover or a time-related index permitting the time of removal to be calculated.

2. A device according to Claim 1 further comprising timing means for recording the time of application of a sample to a receptor (25, 32) associated with a test zone.

3. A device according to either of claims 1 or 2, comprising first and second such zones each containing respective biosensor means and/or reagent media and designated to accept material samples in a predetermined timing sequence, wherein at least the zone designated to accept the later sample is covered until use by a removable humidity resistant cover (16,18).

4. A device according to any preceding claim, wherein respective removable and humidity resistant covers (16, 18) are associated with both of said zones.

5. A device according to any preceding claim wherein removal of a cover (16, 18) is effective to place the device into an operational mode or configuration.

6. A device according to any preceding claim wherein removal of a cover is effective to cancel an alarm indication signalling the need for a procedural step, involving a test zone covered by the cover, to be taken.

7. A device according to any preceding claim wherein a cover is configured to interact with, or to replicate the functionality of, an actuator means intended for manual operation by a user of the device and associated with electronic timing and/or signalling components carried by said device.

8. A device according to any preceding claim, wherein the, or each, humidity resistant cover comprises a shaped cover made of a plastics material, preferably injection-moulded, and adapted to be slidably attached to and removed from the device.

9. A device according to any of claims 1 to 7, wherein the, or each, cover comprises a peel-off strip of material which is self-adhesively secured to the device so as to temporarily cover its respective zone.

10. A device according to any preceding claim, wherein the, or each, cover incorporates a desiccant material.

11. A device according to any preceding claim, wherein the, or each, cover (16, 18) covers not only its respective zone, but also an adjoining portion of the device so as to cover, until removed, certain procedural instructions (23, 24, 30, 31) relating to the conduct of the test.

12. A device according to any preceding claim further comprising means for sensing and recording temperature and/or humidity.

13. A device according to any preceding claim further comprising electronic means for developing electrical signals indicative of a condition of the patient for transfer to a data logging zone (14) for storing and/or further processing said electrical signals.

14. A device according to claim 13, wherein said data logging zone (14) is detachable from the remainder of said device and can be forwarded for analysis of the signals transferred thereto.

15. A device according to any preceding claim further comprising electronic means for processing data relating to said sample or samples and for displaying a result of the test.

## Patentansprüche

1. Biologische Testvorrichtung (10) umfassend zumindest eine Zone, die Biosensormittel und/oder Reaktionsmedien enthält und dazu bestimmt ist, eine Probe eines biologischen Materials aufzunehmen, **dadurch gekennzeichnet, dass**
die Zone bis zur Verwendung durch eine abnehmbare feuchtigkeitsbeständige Abdeckung (16, 18) abgedeckt ist; und die Vorrichtung des Weiteren Zeitnehmungsmittel zur Aufzeichnung des Zeitpunkts des Abnehmens der oder jeder Abdeckung oder einen zeitbezogenen Index umfasst, der die Berechnung des Zeitpunkts des Abnehmens erlaubt.

2. Vorrichtung nach Anspruch 1 des Weiteren umfassend Zeitnehmungsmittel zur Aufzeichnung des Zeitpunkts der Aufbringung einer Probe auf einen Rezeptor (25, 32), der einer Testzone zugeordnet ist.

3. Vorrichtung nach einem der Ansprüche 1 oder 2, umfassend erste und zweite derartige Zonen, die jeweils Biosensormittel und/oder Reaktionsmedien enthalten, und dazu bestimmt sind, Materialproben in einer vorbestimmten zeitlichen Abfolge aufzunehmen, wobei zumindest die Zone, die zur Aufnahme der späteren Probe bestimmt ist, bis zur Verwendung durch eine abnehmbare feuchtigkeitsbeständige Abdeckung (16, 18) abgedeckt ist.

4. Vorrichtung nach einem der vorhergehenden Ansprüche, wobei jeweilige abnehmbare und feuchtigkeitsbeständige Abdeckungen (16, 18) beiden Zonen zugeordnet sind.

5. Vorrichtung nach einem der vorhergehenden Ansprüche, wobei die Abnahme einer Abdeckung (16, 18) bewirkt, dass die Vorrichtung in einen Betriebsmodus oder eine Betriebskonfiguration versetzt wird.

6. Vorrichtung nach einem der vorhergehenden Ansprüche, wobei die Abnahme einer Abdeckung bewirkt, dass eine Alarmangabe abgebrochen wird, die die Notwendigkeit signalisiert, dass ein Verfahrensschritt, der eine mit der Abdeckung abgedeckte Testzone involviert, zu setzen ist.

7. Vorrichtung nach einem der vorhergehenden Ansprüche, wobei eine Abdeckung dazu ausgebildet ist, mit einem Betätigungsmittel zu interagieren oder dessen Funktionalität nachzubilden, das dazu vorgesehen ist, manuell durch einen Benutzer der Vorrichtung betätigt zu werden, und elektronischen Zeitnehmungs- und/oder Signalisierungskomponenten zugeordnet ist, die durch die Vorrichtung getragen werden.

8. Vorrichtung nach einem der vorhergehenden Ansprüche, wobei die oder jede feuchtigkeitsbeständige Abdeckung eine geformte Abdeckung aus einem Kunststoffmaterial umfasst, das vorzugsweise spritzgeformt und dazu geeignet ist, verschiebbar an der Vorrichtung angebracht und davon entfernt zu werden.

9. Vorrichtung nach einem der Ansprüche 1 bis 7, wobei die oder jede Abdeckung einen Abziehstreifen aus einem Material umfasst, das selbstklebend an der Vorrichtung gesichert ist, um die jeweilige Zone temporär abzudecken.

10. Vorrichtung nach einem der vorhergehenden Ansprüche, wobei die oder jede Abdeckung ein Trocknungsmaterial integriert.

11. Vorrichtung nach einem der vorhergehenden Ansprüche, wobei die oder jede Abdeckung (16, 18) nicht nur ihre jeweilige Zone, sondern auch einen angrenzenden Abschnitt der Vorrichtung abdeckt, um so bis zur ihrer Abnahme bestimmte Verfahrensanweisungen (23, 24, 30, 31), die die Durchführung des Tests betreffen, abzudecken.

12. Vorrichtung nach einem der vorhergehenden Ansprüche, des Weiteren umfassend Mittel zur Erfassung und Aufzeichnung der Temperatur und/oder Feuchtigkeit.

13. Vorrichtung nach einem der vorhergehenden Ansprüche, des Weiteren umfassend elektronische Mittel zur Entwicklung elektrischer Signale, die einen Zustand des Patienten angeben, zur Übertragung an eine Datenprotokollierungszone (14) zur Speicherung und/oder weiteren Verarbeitung der elektrischen Signale.

14. Vorrichtung nach Anspruch 13, wobei die Datenprotokollierungszone (14) vom Rest der Vorrichtung ablösbar ist und zur Analyse der darauf übertragenen Signale weitergeleitet werden kann.

15. Vorrichtung nach einem der vorhergehenden Ansprüche, des Weiteren umfassend elektronische Mittel zur Verarbeitung von Daten, die die Probe oder Proben betreffen, sowie zur Anzeige eines Ergebnisses des Tests.

## Revendications

1. Dispositif de test biologique (10) comprenant au moins une zone contenant un moyen de biocapteur et/ou un milieu réactif et désignée pour recevoir un échantillon d'une matière biologique, **CARACTÉRISÉ EN CE QUE**
ladite zone est recouverte jusqu'à son utilisation par un couvercle résistant à l'humidité amovible (16, 18) ; et
ledit dispositif comprend en outre un moyen de synchronisation pour enregistrer le temps de retrait du ou de chaque couvercle ou un indice lié au temps permettant au temps de retrait d'être calculé.

2. Dispositif selon la revendication 1, comprenant en outre un moyen de synchronisation pour enregistrer le temps d'application d'un échantillon à un récepteur (25, 32) associé à une zone de test.

3. Dispositif selon l'une des revendications 1 et 2, comprenant de telles première et deuxième zones contenant chacune un moyen de biocapteur et/ou un milieu réactif respectif(s) et désignée pour recevoir des échantillons de matière dans une séquence de synchronisation prédéterminée, où au moins la zone désignée pour recevoir le dernier échantillon est recouverte jusqu'à son utilisation par un couvercle résistant à l'humidité amovible (16, 18).

4. Dispositif selon l'une des revendications précédentes, dans lequel des couvercles résistant à l'humidité et amovibles respectifs (16, 18) sont associés auxdites deux zones.

5. Dispositif selon l'une des revendications précédentes, dans lequel le retrait d'un couvercle (16, 18) est efficace pour placer le dispositif dans un mode opérationnel ou une configuration opérationnelle.

6. Dispositif selon l'une des revendications précédentes, dans lequel le retrait d'un couvercle est efficace pour annuler une indication d'alarme signalant la nécessité de prendre une mesure de procédure ; impliquant une zone de test recouverte par le couvercle.

7. Dispositif selon l'une des revendications précédentes, dans lequel un couvercle est configuré pour interagir avec, ou pour reproduire la fonctionnalité, d'un moyen d'actionnement destiné à un fonctionnement manuel par un utilisateur du dispositif et associé à des composants électroniques de synchronisation et/ou de signalisation portés par ledit dispositif.

8. Dispositif selon l'une des revendications précédentes, dans lequel le, ou chaque couvercle résistant à l'humidité comprend un couvercle conformé réalisé en une matière plastique, de préférence moulée par injection, et adapté pour être fixé en coulissement au dispositif et être retiré en coulissement de celui-ci.

9. Dispositif selon l'une des revendications 1 à 7, dans lequel le ou chaque couvercle comprend une bande pelable de matière qui est fixée de manière auto-adhésive au dispositif de manière à recouvrir temporairement sa zone respective.

10. Dispositif selon l'une des revendications précédentes, dans lequel le ou chaque couvercle incorpore une matière déshydratante.

11. Dispositif selon l'une des revendications précédentes, dans lequel le ou chaque couvercle (16, 18) recouvre non seulement sa zone respective, mais également une partie contiguë du dispositif de manière à recouvrir, jusqu'à ce qu'il soit retiré, certaines instructions de procédure (23, 24, 30, 31) liées au déroulement du test.

12. Dispositif selon l'une des revendications précédentes, comprenant en outre un moyen pour détecter et enregistrer la température et/ou l'humidité.

13. Dispositif selon l'une des revendications précédentes, comprenant en outre un moyen électronique pour développer des signaux électriques indiquant un état du patient pour les transférer à une zone d'enregistrement de données (14) pour stocker et/ou traiter davantage lesdits signaux électriques.

14. Dispositif selon la revendication 13, dans lequel ladite zone d'enregistrement de données (14) est détachable du reste dudit dispositif et peut être envoyée pour l'analyse des signaux transférés à celle-ci.

15. Dispositif selon l'une des revendications précédentes, comprenant en outre un moyen électronique pour traiter des données liées audit échantillon ou auxdits échantillons et pour afficher un résultat du test.
